# DEMANDE DE BREVET EUROPEEN

(11) **EP 1 245 300 A1**
(43) Date de publication de la demande: **02.10.2002**
(21) Numéro de dépôt: 02370009.9
(22) Date de dépôt: 27.03.2002
(51) Int. Cl.: B08B 9/00, B08B 3/00, C11D 11/00

(54) **Composition de produit de nettoyage pour circuit de chauffage ou circuit de climatisation**

(30) Priorité: 30.03.2001 FR 0104406
(71) Demandeur: Sadaps-Bardahl Corporation SA, 59115 Leers (FR)
(72) Inventeur: Rigot, Gilles, 59510 Hem (FR); Pietruszka, Alfred, 62110 Henin Beaumont (FR)
(74) Mandataire: Hennion, Jean-Claude

(57) **Abrégé**

La présente invention concerne une composition de produit de nettoyage pour circuit de chauffage ou de climatisation qui comprend un biocide et un solvant. De manière caractéristique selon l'invention le solvant contient majoritairement un alcool ou un mélange d'au moins deux alcools.

## Description

La présente invention concerne une composition de produit de nettoyage pour circuit de chauffage ou de climatisation, notamment pour les circuits de chauffage ou de climatisation des véhicules tels que les automobiles, les véhicules poids lourds, les engins agricoles ou de travaux publiques ou tout autre type de véhicule.

Le circuit de chauffage d'un véhicule comprend une entrée d'air disposée au niveau du moteur, un ventilateur qui assure la circulation de l'air depuis l'extérieur du véhicule jusque dans l'habitacle et un radiateur qui permet de chauffer l'air entrant dans le circuit avant son entrée dans l'habitacle. Dans le cas d'un système de climatisation, le circuit comprend également un évaporateur disposé en amont du radiateur et qui permet de refroidir l'air entrant. Un système de pales permet, en fonction de la température désirée à l'intérieur de l'habitacle, de faire circuler tout ou partie de l'air provenant de l'évaporateur, soit vers le radiateur en sorte de le réchauffer, soit vers l'habitacle. La prolifération bactérienne est très importante, plus particulièrement, dans le cas d'un système de climatisation, notamment au niveau de l'évaporateur qui produit de la vapeur d'eau. Cette vapeur d'eau contenue dans l'air se condense ensuite lors du refroidissement. Cette eau condensée stagne dans les conduits du circuit et favorise donc la prolifération des bactéries véhiculées par l'air entrant dans le circuit et les poussières qu'il transporte. Cette prolifération bactérienne se répand peu à peu de l'évaporateur à toutes les parois des conduits du système. Les bactéries sont ensuite propulsées dans l'habitacle causant ainsi des maladies et/ou des allergies des voies aériennes chez les occupants.

Les produits de nettoyage pour circuit de chauffage ou de climatisation de véhicules sont composés d'un agent biocide dilué dans un solvant contenant majoritairement de l'eau additionnée éventuellement de tensioactifs. Le produit de nettoyage est en général conditionné dans un atomiseur en sorte d'être injecté par pulvérisation dans les conduits du système de chauffage ou de climatisation via l'entrée d'air du circuit qui est accessible depuis l'extérieur ou l'intérieur de l'habitacle selon le type de véhicule.

La présente invention propose une nouvelle composition pour un produit de nettoyage pour circuit de chauffage ou de climatisation qui comprend, de manière connue un biocide et un solvant. De manière caractéristique selon l'invention, le solvant contient majoritairement un alcool ou un mélange d'au moins deux alcools. La composition de l'invention n'est pas limitée au nettoyage des circuits de chauffage ou de climatisation de véhicules; elle peut également être utilisée pour le nettoyage de circuits de chauffage ou de climatisation installés, par exemple, dans des immeubles.

Du fait de l'utilisation d'un solvant majoritairement alcoolique, la composition de l'invention s'évapore rapidement même à basse température ce qui évite tout dégagement de vapeur d'eau lors d'une mise en fonctionnement du système de climatisation après un nettoyage avec le produit de l'invention. Ce dégagement de vapeur qui est généré après nettoyage avec un produit dont le solvant contient majoritairement de l'eau s'avère très gênant pour l'utilisateur dans le cas d'un espace réduit tel que l'habitacle d'un véhicule, comme par exemple, une automobile, la vapeur d'eau envahissant tout l'habitacle au plus grand désagrément des passagers.

Avantageusement, le solvant contient entre 75% et 100% d'alcool(s).

L'alcool ou les alcools utilisés ne sont pas limités selon l'invention. Il est ainsi possible d'utiliser l'éthanol, le propanol, le butanol, le pentanol et l'hexanol.

De même, l'agent biocide n'est pas limité selon l'invention. Tout biocide connu est susceptible d'être soluble dans un alcool ou un mélange d'alcools peut ainsi être utilisé.

Selon un mode de réalisation particulier, l'agent biocide contient de l'isothiazolone. Un tel agent biocide s'est avéré être remarquable tant du point de vue curatif que préventif. En effet, cet agent biocide permet d'éliminer rapidement, en quelques minutes, tous les micro-organismes présents dans le système de chauffage ou de climatisation mais il permet également de former un film biocide actif empêchant pendant une très longue durée, de l'ordre de 6 mois à 12 mois, selon l'utilisation du chauffage ou de la climatisation et le nombre de filtres disposés dans le circuit, la prolifération bactérienne et microbienne dans le système de chauffage ou de climatisation traité avec le produit de l'invention ce qui permet d'espacer les nettoyages. Le spectre d'action de l'agent biocide précité est très large et permet d'éliminer l'ensemble des microorganismes présents dans le système, tels que par exemple, les bactéries et les champignons.

Selon un mode de réalisation particulier de la composition de l'invention, la proportion en agent biocide est comprise entre 2% et 10%.

De même selon un mode de réalisation de la présente invention, la composition contient en outre un agent anti-corrosion qui permet lors du nettoyage du système de chauffage ou de climatisation de traiter les parties nettoyées et susceptibles d'être corrodées contre la corrosion.

La proportion en agent anti-corrosion est par exemple, comprise entre 0.3 et 1,5%.

Tout agent anti-corrosion connu peut être utilisé. Ainsi, selon une variante particulière de réalisation, l'agent anti-corrosion se présente sous la forme d'une cire. Un tel agent anti-corrosion permet de former un film uniforme contenant le biocide. De ce fait, le biocide est réparti uniformément sous forme de film dans le système de chauffage ou de climatisation assurant ainsi des propriétés bio-statiques particulièrement satisfaisantes. Par ailleurs, la formation du film précité permet de recouvrir, d'une part, les poussières déposées sur les parois des conduits, évitant ainsi qu'elles ne soient respirées par le conducteur du véhicule et d'autre part les particules de graisse déposées sur les parois de ces mêmes conduits et qui constituent des sites favorables à la prolifération bactérienne et à l'accumulation de poussières. Ainsi, la composition de l'invention remplit plusieurs fonctions : elle permet d'éliminer les bactéries qui sont susceptibles à la fois d'être une source d'allergies et/ou de maladies et une source d'odeurs désagréables, de traiter les poussières contenues dans l'air en sorte de les désinfecter, de traiter toutes les parois du circuit en formant un film biocide actif présentant une activité longue durée, ce film permettant de plus de neutraliser les poussières déposées et de recouvrir les particules de graisse comme précédemment indiqué.

Selon une variante de réalisation, l'agent anti-corrosion contient du dioléate de N-alkyl-oleylpropylène diamine; un tel agent anti-corrosion se présente sous la forme d'une cire.

La présente invention concerne également un produit de nettoyage pour circuit de chauffage ou de climatisation qui contient la composition de l'invention.

Ce produit peut être conditionné dans un flacon dont au moins les parois internes sont en aluminium, ce qui permet d'éviter toute réaction chimique entre la composition de l'invention et le matériau des parois. Le produit de l'invention peut ainsi être conservé pendant une très longue durée sans perdre de son efficacité et sans apparition de dépôts provenant de l'attaque des parois internes du container et qui sont nuisibles à la vaporisation du produit.

La présente invention concerne également un procédé de nettoyage d'un circuit de chauffage ou de climatisation, selon lequel on fait circuler l'air dans le circuit de chauffage ou de climatisation et on introduit sous forme de brume la composition de l'invention dans le circuit, de préférence par l'orifice d'entrée d'air du circuit, l'air circulant dans le circuit. L'introduction de la composition de l'invention sous forme de brume permet de former sur toutes les parois des conduits du circuit et de manière uniforme, le film biocide précité et donc de traiter l'intégralité du circuit. La propagation de la brume est assurée par la circulation de l'air dans le circuit.

La présente invention, ses caractéristiques et les différents avantages qu'elle procure apparaîtront mieux à la lecture de la description qui suit d'un exemple de composition selon l'invention.

Les différents composants de l'exemple de composition selon l'invention sont résumés dans le tableau I ci-dessous.

**TABLEAU I**

| composant | % en masse |
|---|---|
| ethanol | 93,9 |
| agent biocide | 5,0 |
| agent anti-corrosion | 0,9 |
| parfum (citron) | 0,2 |

On notera que la composition de l'invention et notamment la composition selon l'exemple précité n'est pas moussante ce qui la rend facile à utiliser car elle se disperse aisément, uniformément et rapidement dans les conduits d'un système de chauffage ou de climatisation quel qu'il soit et, en particulier, dans un système de chauffage ou de climatisation d'automobile, sans les obstruer.

Cette composition peut être utilisée pour la fabrication d'un produit de nettoyage pour circuit de chauffage ou de climatisation et plus particulièrement pour un circuit de chauffage ou de climatisation de véhicule. La composition est alors conditionnée dans un pulvérisateur susceptible d'être équipé d'un tube prolongateur facile à insérer à travers l'entré d'air du circuit. Le pulvérisateur est de préférence du type pouvant être utilisé dans n'importe quelle position °C.

## Revendications

1. Composition de produit de nettoyage pour circuit de chauffage ou de climatisation qui comprend un biocide et un solvant **caractérisée en ce que** ledit solvant contient majoritairement un alcool ou un mélange d'au moins deux alcools.

2. Composition de produit de nettoyage selon la revendication 1, **caractérisée en ce que** ledit solvant contient entre 75% et 100% dudit alcool ou dudit mélange d'alcools.

3. Composition de produit de nettoyage selon la revendication 1 ou 2, **caractérisée en ce que** ledit alcool ou les deux alcools dudit mélange sont choisis parmi l'éthanol, le propanol, le butanol, le pentanol et l'hexanol.

4. Composition de produit de nettoyage selon l'une quelconque des revendications 1 à 3, **caractérisée en ce que** ledit biocide contient de l'isothiazolone.

5. Composition selon l'une quelconque des revendications 1 à 4, **caractérisée en ce qu'**elle contient entre 2% et 10% d'agent biocide.

6. Composition de produit de nettoyage selon l'une quelconque des revendications 1 à 5, **caractérisée en ce qu'**elle contient en outre un agent anti-corrosion.

7. Composition selon la revendication 6, **caractérisée en ce que** ledit agent anti-corrosion est une cire.

8. Composition selon la revendication 6 ou 7, **caractérisée en ce que** la proportion en agent anti-corrosion est comprise entre 0.3 et 1,5%

9. Composition de produit de nettoyage selon l'une quelconque des revendications 6 à 8, **caractérisée en ce que** ledit agent anti-corrosion contient du dioléate de N-alkyl-oleylpropylène diamine.

10. Composition selon l'une quelconque des revendications 1 à 10, **caractérisée en ce qu'**elle contient en outre un parfum.

11. Produit de nettoyage pour circuit de chauffage ou de climatisation, **caractérisé en ce qu'**il contient la composition selon l'une quelconque des revendications 1 à 10.

12. Produit de nettoyage pour circuit de chauffage ou de climatisation selon la revendication 11, **caractérisé en ce qu'**il est conditionné dans un flacon dont au moins les parois internes sont en aluminium.

13. Procédé de nettoyage d'un circuit de chauffage ou de climatisation, **caractérisé en ce que** :
- on fait circuler l'air dans le circuit de chauffage ou de climatisation ; et
- on introduit sous forme de brume la composition selon l'une quelconque des revendications 1 à 10 dans ledit circuit, de préférence par l'orifice d'entrée d'air du circuit, l'air circulant dans ledit circuit.
